# EUROPÄISCHE PATENTSCHRIFT

(11) **EP 1 627 622 B1**
(45) Veröffentlichungstag und Bekanntmachung des Hinweises auf die Patenterteilung: **10.09.2014**
(21) Anmeldenummer: 05106290.9
(22) Anmeldetag: 11.07.2005
(51) Int. Cl.: A61Q 19/10, A61K 8/46, A61K 8/44, A61K 8/81

(54) **Reinigungsgel**
Cleansing gel
Gel nettoyant

(30) Priorität: 14.07.2004 DE 102004034915
(43) Veröffentlichungstag der Anmeldung: 22.02.2006
(73) Patentinhaber: Beiersdorf AG, 20253 Hamburg (DE)
(72) Erfinder: Dörschner, Albrecht, 20146, Hamburg (DE); Racine, Magalie, 22089, Hamburg (DE); Ruppert, Stephan, 20259 Hamburg (DE); Kohut, Michaela, 20257, Hamburg (DE)

(56) Entgegenhaltungen:
- EP-A- 1 275 371
- EP-A- 1 470 813
- WO-A-03/030807
- WO-A-03/097005
- WO-A-2004/006882
- WO-A2-02/05758
- 'Haircare Range - Shampoo' 01 Juli 2002, XP055002422

## Beschreibung

Die vorliegende Erfindung betrifft ein kosmetisches Reinigungsgel enthaltend Myrethsulfat, Cocamidopropylbetain, quervernetztes Acrylat-Copolymer und Wasser.

Der Wunsch nach sauberer Haut ist wohl so alt wie die Menschheit, denn Schmutz, Schweiß und Reste abgestorbener Hautpartikel bieten den idealen Nährboden für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neu entwickelten synthetischen Tensiden formuliert werden konnten. Baden und Duschen sind seitdem aus unserem täglichen Leben nicht mehr wegzudenken. Den Verbrauchern stehen heutzutage eine Vielzahl von Produkten für die Reinigung der verschiedenen Körperpartien zur Verfügung.

Eine besondere Gruppe an Hautreinigungsprodukten bilden dabei die Gesichtsreinigungsprodukte. Da die Gesichtshaut besonders empfindlich ist, werden für die Gesichtsreinigung besonders milde und die Haut nicht reizende Produkte eingesetzt. Meist werden dabei Gele, das heißt halbfeste, mehr oder weniger transparente Systeme verwendet.

Reinigungsgele enthalten als Hauptbestandteile Wasser, Tenside und Verdicker (Gelbildner).

Die Tenside stellen in den Reinigungsgelen die waschaktive Substanzen dar. Sie sorgen, bedingt durch ihren spezifischen Molekülaufbau mit jeweils einer hydrophilen (wasseranziehenden) und hydrophoben (wasserabweisenden) Gruppierung im gleichen Molekül, für die Herabsetzung der Oberflächenspannung des Wassers, wodurch die Schmutzentfernung erleichtert wird. Man unterscheidet je nach Ladungszustand zwischen anionischen, kationischen, amphoteren und nichtionischen Tensiden. Aufgrund ihrer Fähigkeit, die Oberflächespannung von Wasser zu reduzieren, bewirken Tenside ein Aufschäumen der Zubereitung.

Die Verdicker, auch Gelbildner genannt, bilden in dem Reinigungsgel ein dreidimensionales Netzwerk aus, in dem die Flüssigkeit (in der Regel Wasser) immobilisiert ist. Als Verdicker werden neben Salzen meist Polymere wie Polyacrylate eingesetzt. Diese werden der zu verdickenden Zubereitung bei neutralem pH-Wert zugesetzt und anschließend durch die Zugabe von Basen deprotoniert, wodurch die Zubereitung in ein viskoses Gel übergeht.

Grundsätzlich sollen Reinigungsgele sowohl mild und schonend zur Haut sein als auch einen feinporigen, cremigen Schaum in ausreichender Menge erzeugen. Ferner sollen sie möglichst transparent und klar sein, da sich so wirkungsvoll optische Effektstoffe einarbeiten lassen und transparente Zubereitungen vom Verbraucher aus ästhetischen Gründen bevorzugt werden.

Herkömmliche, nach dem Stand der Technik hergestellte Reinigungsgele weisen eine Reihe von Unzulänglichkeiten auf:
- Damit die Zubereitung mild wird und die Haut nicht zu stark entfettet, dürfen nur ausgewählte Tenside in geringer Konzentration in die Zubereitung eingearbeitet werden. Derartige Zubereitungen lassen sich aber nur unzureichend aufschäumen. Der Schaum ist nicht besonders feinporig und cremig und fällt schnell wieder in sich zusammen. Auch lässt sich die Viskosität von Zubereitungen mit einem geringen Tensidgehalt nicht auf herkömmliche Art und Weise durch den Zusatz von Salzen erhöhen.
- Um die Viskosität von Zubereitungen mit geringem Tensidgehalt zu erhöhen, müssen der Formulierung Polymere Verdicker (z.B. Carbopole) zugesetzt werden. Derartige Verdicker behindern aber die Schaumbildung der Zubereitung.
- Die Kombination aus herkömmlichen Tensiden und polymeren Verdickern führt in der Regel zur Eintrübung der Zubereitung. Optische Effekte, beispielsweise der Zusatz von Abrasiva oder Glitterstoffen, kommen nur unzureichend zur Geltung.

Es war daher die Aufgabe der vorliegenden Erfindung, die Mängel des Standes der Technik zu beseitigen und milde Reinigungsgele mit großer Schaumkraft und hoher Transparenz zu entwickeln.

Überraschend gelöst werden die Aufgaben durch ein kosmetisches Reinigungsgel enthaltend
a) 1-5 Gewichts-% Myrethsulfat,
b) 1,5-7 Gewichts-% Cocamidopropylbetain,
c) 2-10 Gewichts-% quervernetztes Acrylat-Copolymer,
d) 70 bis 95 Gewichts-% Wasser,
jeweils bezogen auf das Gesamtgewicht der Zubereitung,
dadurch gekennzeichnet, dass das Gewichtsverhältnis von Myrethsulfat zu Cocamidopropylbetain von 0,1:1 bis 1,3:1 beträgt und die Zubereitung eine Viskosität von 1500-10000 mPas aufweist.

Die erfindungsgemäße Viskosität wurde mit Hilfe eines Viskotesters VT 02 der Gesellschaft Haake (Temperatur: 25 °C, Spindel: Drehkörper 1 (24 mm Durchmesser), Rotorgeschwindigkeit: 62,5 min⁻¹) ermittelt.

Zwar kennt der Stand der Technik Reinigungsgele enthaltend quervernetzte AcrylatCopolymere. So beschreiben die WO 01/076552, WO 01/019946, EP 1291000, EP 1291001, EP1291002, EP1291003 und EP 1291005 derartige Zubereitungen. Darüber hinaus kennt der Fachmann die WO 03/097005, WO 03/030807, WO 2004/006882, XP55002422 und WO 02/05758. Doch konnten diese Schriften nicht den Weg zur vorliegenden Erfindung weisen.

Es ist erfindungsgemäß bevorzugt, wenn die Konzentration an Myrethsulfat im Reinigungsgel von 1,5 bis 3 Gewichts-%, bezogen auf das Gesamtgewicht der Zubereitung beträgt.

Erfindungsgemäß bevorzugte Myrethsulfate sind insbesondere die Verbindungen: Texapon K 14 S Spezial (Cognis), CASR-Nummer 68891-38-4, Standapol ES 40 (Cognis), CASR-Nummer 68585-34-2 und Zetesol 470 (Zschimmer&Schwarz), CAS-Nummer 25446-80-4.

Es ist erfindungsgemäß bevorzugt, wenn die Konzentration an Cocamidopropylbetain im Reinigungsgel von 2 bis 5 Gewichts-%, bezogen auf das Gesamtgewicht der Zubereitung beträgt.

Erfindungsgemäß ist Cocamidopropylbetain dabei die INCI-Bezeichnung für Cocamidopropyldimethylglycin, welches unter der CAS-Nummer 61789-40-0 (I) abgelegt ist.

Erfindungsgemäß bevorzugt beträgt das Gewichtsverhältnis von Myrethsulfat zu Cocamidopropylbetain ungefähr 0,5:1.

Erfindungsgemäß bevorzugt wird eine Verbindung, die von der Firma Noveon unter der Bezeichnung Carbopol Aqua SF-1 angeboten wird, eingesetzt. Dieses stellt ein leicht quervernetztes durch Alkalien quellbares Acrylat-Copolymer dar, welches drei Strukturkomponenten enthält, nämlich eine oder mehrere Carboxylsäuremonomere mit 3 bis 10 Kohlenstoffatomen, ein oder mehrere Vinylmonomere sowie als dritte Komponente ein-oder mehrfach ungesättigte Monomere.

Das erfindungsgemäße, quervernetzte Acrylat-Copolymer wird erfindungsgemäß bevorzugt in einer Konzentration von 3-7 Gewichts-%, bezogen auf das Gesamtgewicht der Zubereitung, in das Reinigungsgel eingearbeitet.

Die erfindungsgemäße Transmission wurde erfindungsgemäß mit dem Gerät Agilent 8453 Diode Array Spectrometer bei einer Schichtdicke von 1 cm bestimmt. Die Messung wurde in einem Bereich von 190 bis 1100 nm in Intervallen von 1 nm und einer Integrationszeit von 0,5 Sekunden bei Raumtemperatur durchgeführt. Erfindungsgemäß bevorzugt beträgt die Transmission der erfindungsgemäßen Zubereitung von 50% bis 100% bei 420 nm Wellenlänge.

Auch ist es erfindungsgemäß vorteilhaft, wenn das Reinigungsgel einen pH-Wert von pH 6 bis pH 8 aufweist.

Erfindungsgemäß bevorzugte Ausführungsformen der vorliegenden Erfindung sind dadurch gekennzeichnet, dass die Zubereitung einen oder mehrere Konservierungsstoffe in einer Konzentration von 0,1 bis 2,0 Gewichts-% und besonders bevorzugt von 0,2 bis 1,2 Gewichts-%, jeweils bezogen auf das Gesamtgewicht der Zubereitung, enthält.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Erfindungsgemäß besonders bevorzugt ist dabei der Einsatz von Parabenen und Phenoxyethanol als Konservierungsmittel.

Vorteilhaft im Sinne der vorliegenden Erfindung ist es, wenn das erfindungsgemäße Reinigungsgel einen oder mehrere Komplexbildner in einer Gesamtkonzentration von 0,1 bis 2,0 Gewichts-% und besonders bevorzugt in einer Konzentration von 0,2 bis 1,0 Gewichts-%, jeweils bezogen auf das Gesamtgewicht der Zubereitung, enthält.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Erfindungsgemäß besonders bevorzugt ist es Hydroxyethylendiaminotriessigsäure und insbesondere das Natriumsalz der Hydroxyethylendiaminotriessigsäure (Na₃HEDTA) als Komplexbildner einzusetzen.

Es ist erfindungsgemäß vorteilhaft, wenn das erfindungsgemäße Reinigungsggel einen oder mehrere Hautbefeuchtungsmittel in einer Konzentration von 0,5 bis 10 Gewichts-% und bevorzugt in einer Konzentration von 2,0 bis 6,0 Gewichts-%, jeweils bezogen auf das Gesamtgewicht der Zubereitung, enthält.

Erfindungsgemäß vorteilhafte Hautbefeuchtungsmittel sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Erfindungsgemäß besonders bevorzugt ist dabei der Einsatz von Glycerin als Hautbefeuchtungsmittel.

Es ist erfindungsgemäß vorteilhaft, wenn das erfindungsgemäße Reinigungsgel Abrasiva (z.B. Peelingpartikel aus Polyethylen), Glitterstoffe, Effektstoffe, Farbschlieren, Gasblasen (insbesondere Luftblasen), Perlglanzpigmente, Glimmer, Öl- und/oder Emulsionströpchen enthält.

Auch ist es vorteilhaft im Sinne der vorliegenden Erfindung, wenn das erfindungsgemäße Reinigungsgel einen oder mehrere Farbstoffe enthält. Dabei können insbesondere alle bekannten, wasserlöslichen und für die Kosmetik zugelassenen Farbstoffe der Zubereitung zugesetzt werden.

Selbstverständlich ist es erfindungsgemäß von Vorteil, dem erfindungsgemäßen Reinigungsgel weitere kosmetische Wirk-, Hilfs- und Zusatzstoffe zuzusetzen. Die Nachfolgende Aufzählung gibt eine kleine Auswahl erfindungsgemäß vorteilhafter weiterer Zusätze, die aber keinesfalls die vorliegende Erfindung auf diese Verbindungen beschränken soll.

Die wässrige Phase der erfindungsgemäßen Reinigungsgele kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Perlglanzagentien, Antischuppenwirkstoffe, Pflanzenextrakte, Vitamine, Wirkstoffe.

Besonders vorteilhafte Zubereitungen werden ferner erhalten, wenn als Zusatz- oder Wirkstoffe Antioxidantien eingesetzt werden.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid), Licochalcon A, Ascorbinsäure und deren Derivate Vitamin E und dessen Derivate, Vitamin A und dessen Derivate,γ-Oryzanol, Panthenol und/oder Niacinamid.

Ein weiterer erfindungsgemäß vorteilhafter Wirkstoff stellt beispielsweise Polidocanol dar.

Die Menge der Wirkstoffe (eine oder mehrere Verbindungen) in den Zubereitungen beträgt erfindungsgemäß vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Erfindungsgemäß vorteilhaft wird die erfindungsgemäße Zubereitung in einer Tube, einem Pumpspender oder einer Flasche aufbewahrt und aus dieser heraus angewendet. Derartige Verpackungsbehältnisse sind erfindungsgemäß bevorzugt transparent.

Daher sind auch Tuben, Pumpspender oder Flaschen enthaltend eine erfindungsgemäße Zubereitung, erfindungsgemäß, wobei transparente Verpackungsbehältnisse erfindungsgemäß bevorzugt sind.

Vorteilhaft im Sinne der vorliegenden Erfindung ist es ferner, das erfindungsgemäße Reinigungsgel auf ein Trägermaterial (z.B. ein Tuch, Pad, Wattebausch) aufzutragen. Derartige Träger bestehen vorzugsweise aus Viskose-, Baumwolle- und/oder Polyesterfasern.

Erfindungsgemäß ist daher auch ein Trägermaterial (z.B. ein Tuch, Pad, Wattebausch) getränkt mit einem erfindungsgemäßen Reinigungsgel, wobei das Trägermaterial bevorzugt aus Viskose-, Baumwolle- und/oder Polyesterfasern aufgebaut ist.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

| | **1** | **2** | **3** | **4** | **5** |
|---|---|---|---|---|---|
| Natriummyrethsulfat | 5% | 4% | 3% | 3% | 3% |
| Cocoamidopropylbetain | 7% | 5% | 4% | 2% | 1,5% |
| Laurylglucoside | -- | 1% | -- | 1% | 2% |
| Acrylates Copolymer | 4% | 5% | 4% | 3% | 7% |
| Natriumhydroxid | 0,5% | 0,5% | 0,5% | 0,5% | 0,5% |
| Glycerin | 5% | 10% | 2% | 10% | --- |
| Polyquaternium-10 | 0,1% | -- | 0,2% | -- | 0,3% |
| Na₃HEDTA | 0,5% | 0,5% | 0,5% | 0,5% | 0,5% |
| Polyethylen | 1% | 1,5% | 2% | -- | -- |
| PEG-40 hydriertes Rizinusöl | - | 1% | 0,5% | 1% | -- |
| Phenoxyethanol | 0,5% | 0,5% | 0,5% | 0,5% | 0,5% |
| Parabene | 0,2% | 0,2% | 0,2% | 0,2% | 0,2% |
| Parfum | q.s. | q.s. | q.s. | q.s. | q.s. |
| Wasser | ad 100 | ad 100 | ad 100 | ad 100 | ad 100 |

## Patentansprüche

1. Kosmetisches Reinigungsgel enthaltend
a) 1-5 Gewichts-% Myrethsulfat,
b) 1,5-7 Gewichts-% Cocamidopropylbetain,
c) 2-10 Gewichts-% quervernetztes Acrylat-Copolymer,
d) 70 bis 95 Gewichts-% Wasser,
jeweils bezogen auf das Gesamtgewicht der Zubereitung,
**dadurch gekennzeichnet, dass** das Gewichtsverhältnis von Myrethsulfat zu Cocamidopropylbetain von 0,1:1 bis 1,3:1 beträgt und die Zubereitung eine Viskosität von 1500-10000 mPas bei 25°C aufweist.

2. Reinigungsgel nach Anspruch 1, **dadurch gekennzeichnet, dass** es eine Transmission von 50% bis 100% bei einer Wellenlänge von 420 nm aufweist.

3. Reinigungsgel nach einem der vorhergehenden Ansprüche, **dadurch gekennzeichnet, dass** die Zubereitung einen oder mehrere Konservierungsstoffe in einer Konzentration von 0,1 bis 2,0 Gewichts-%, bezogen auf das Gesamtgewicht der Zubereitung, enthält.

4. Reinigungsgel nach einem der vorhergehenden Ansprüche, **dadurch gekennzeichnet, dass** die Zubereitung einen oder mehrere Komplexbildner in einer Gesamtkonzentration von 0,1 bis 2,0 Gewichts-%, bezogen auf das Gesamtgewicht der Zubereitung, enthält.

5. Reinigungsgel nach einem der vorhergehenden Ansprüche, **dadurch gekennzeichnet, dass** die Zubereitung einen oder mehrere Hautbefeuchtungsmittel in einer Konzentration von 0,5 bis 10,0 Gewichts-%, bezogen auf das Gesamtgewicht der Zubereitung, enthält.

6. Reinigungsgel nach einem der vorhergehenden Ansprüche, **dadurch gekennzeichnet, dass** die Zubereitung einen oder mehrere Farbstoffe enthält.

7. Reinigungsgel nach einem der vorhergehenden Ansprüche, **dadurch gekennzeichnet, dass** die Zubereitung Abrasiva, Glitterstoffe, Gasblasen, Effektstoffe, Farbschlieren, Öl- und/oder Emulsionströpchen enthält.

8. Tube, Pumpspender oder Flasche enthaltend eine Zubereitung nach einem der vorhergehenden Ansprüche.

9. Verpackungsbehältnis nach Anspruch 8, **dadurch gekennzeichnet, dass** das Verpackungsbehältnis transparent ist.

10. Trägermaterial getränkt mit einer Zubereitung nach einem der vorhergehenden Ansprüche.

## Claims

1. Cosmetic cleansing gel comprising
a) 1-5% by weight of myreth-sulphate,
b) 1.5-7% by weight of cocamidoprapylbetaine,
c) 2-10% by weight of crosslinked acrylate copolymer,
d) 70 to 95% by weight of water,
in each case based on the total weight of the preparation,
**characterized in that** the weight ratio of myreth-sulphate to cocamidopropylbetaine is from 0.1:1 to 1,3:1 and the preparation has a viscosity of 1500-10 000 mPas at 25°C.

2. Cleansing gel according to Claim 1, **characterized in that** it has a transmission of 50% to 100% at a wavelength of 420 nm.

3. Cleansing gel according to one of the preceding claims, **characterized in that** the preparation comprises one or more preservatives in a concentration of 0.1 to 2.0% by weight, based on the total weight of the preparation.

4. Cleansing gel according to one of the preceding claims, **characterized in that** the preparation comprises one or more complexing agents in a total concentration of 0.1 to 2.0% by weight, based on the total weight of the preparation.

5. Cleansing gel according to one of the preceding claims, **characterized in that** the preparation comprises one or more skin moisturizers in a concentration from 0.5 to 10.0% by weight, based on the total weight of the preparation.

6. Cleansing gel according to one of the preceding claims, **characterized in that** the preparation comprises one or more dyes.

7. Cleansing gel according to one of the preceding claims, **characterized in that** the preparation comprises abrasives, glitter substances, gas bubbles, effect substances, coloured streaks, oil and/or emulsion droplets.

8. Tube, pump dispenser or bottle containing a preparation according to one of the preceding claims.

9. Packaging container according to Claim 8, **characterized in that** the packaging container is transparent.

10. Carrier material impregnated with a preparation according to one of the preceding claims.

## Revendications

1. Gel nettoyant cosmétique, contenant
a) 1-5 % en poids de myreth sulfate,
b) 1,5-7 % en poids de cocoamidopropylbétaine,
c) 2-10 % en poids de copolymère d'acrylate réticulé,
d) 70 à 95 % en poids d'eau,
chaque fois par rapport au poids total de la préparation,
**caractérisé en ce que** le rapport pondéral du myreth sulfate à la cocoamidopropylbétaïne vaut de 0,1:1 à 1,3:1 et la préparation présente une viscosité de 1 500 - 10 000 mPa.s à 25 °C.

2. Gel nettoyant selon la revendication 1, **caractérisé en ce qu'**il présente une transmission de 50 % à 100 % à une longueur d'onde de 420 nm.

3. Gel nettoyant selon l'une quelconque des revendications précédentes, **caractérisé en ce que** la préparation contient en ou plusieurs conservateurs à une concentration de 0,1 à 2,0 % en poids, par rapport au poids total de la préparation.

4. Gel nettoyant selon l'une quelconque des revendications précédentes, **caractérisé en ce que** la préparation contient un ou plusieurs complexants à une concentration totale de 0,1 à 2,0 % en poids, par rapport au poids total de la préparation.

5. Gel nettoyant selon l'une quelconque des revendications précédentes, **caractérisé en ce que** la préparation contient un ou plusieurs agents hydratants pour la peau à une concentration de 0,5 à 10,0 % en poids, par rapport au poids total de la préparation.

6. Gel nettoyant selon l'une quelconque des revendications précédentes, **caractérisé en ce que** la préparation contient un ou plusieurs colorants.

7. Gel nettoyant selon l'une quelconque des revendications précédentes, **caractérisé en ce que** la préparation contient des abrasifs, des paillettes, des bulles de gaz, des substances à effet, des stries colorées, des gouttelettes d'huile et/ou d'émulsion.

8. Tube, distributeur à pompe ou flacon, contenant une préparation selon l'une quelconque des revendications précédentes.

9. Récipient de conditionnement selon la revendication 8, **caractérisé en ce que** le récipient de conditionnement est transparent.

10. Matériau de support imprégné avec une préparation selon l'une quelconque des revendications précédentes.
